# EUROPEAN PATENT SPECIFICATION

(11) **EP 1 221 927 B1**
(45) Date of publication and mention of the grant of the patent: **28.07.2010**
(21) Application number: 00970662.3
(22) Date of filing: 06.10.2000
(51) Int. Cl.: A61K 8/02, A61K 8/84, A61Q 19/00, A61K 9/70

(54) **ELECTROSPUN SKIN MASKS AND USES THEREOF**
GESICHTSMASKE AUS ELEKTROGESPONNENEN FASERN UND IHRE VERWENDUNG
MASQUES DERMATOLOGIQUES ELECTROSTATIQUEMENT FILES ET LEURS UTILISATIONS

(30) Priority: 08.10.1999 US 158674 P
(43) Date of publication of application: 17.07.2002
(73) Proprietor: The University of Akron, Akron Ohio 44325 (US)
(72) Inventor: SMITH, Daniel, Stow, OH 44224 (US); RENEKER, Darrell, Akron, OH 44313 (US); KATAPHINAN, Woraphon, Akron, OH 44311 (US); DABNEY, Sally, Akron, OH 44301 (US)
(74) Representative: Makovski, Priscilla Mary
(86) International application number: PCT/US2000/027775
(87) International publication number: WO 2001/026610

(56) References cited:
- WO-A-98/03267
- US-A- 4 043 331
- US-A- 4 074 366
- US-A- 5 425 938
- US-A- 5 844 017
- DATABASE WPI Derwent Publications Ltd., London, GB; AN 1996-018586 XP002313176 & RU 2 034 534 C (EKOMEDSERVIS CO LTD) 10 May 1995 (1995-05-10) & RU 2 034 534 C (TOVARISHCHESTVO S OGRANICHENNO) 10 May 1995 (1995-05-10)

## Description

### TECHNICAL FIELD

This invention relates to a skin care product and, more particularly, to a cosmetic skin care mask for the treatment of any of a number of skin conditions wherein additives added to the skin are likely to aid in such treatment or wherein the absorption of excess moisture or oils from the skin is likely to aid in such treatment. Specifically, the present invention relates to a skin care mask produced by electrospinning polymeric material, with or without various additives having skin healing, skin cleansing, or other therapeutical or medical properties, into one or more fibers to be applied or deposited directly to the skin, preferably without the use of any intermediate fabrication steps. Once the function of the additives or the fibers have been performed, the mask may be peeled or otherwise removed from the skin.

### BACKGROUND OF THE INVENTION

The technique of electrospinning, also known within the fiber forming industry as electrostatic spinning, of liquids and/or solutions capable of forming fibers, is well known and has been described in a number of patents as well as in the general literature.

The process of electrospinning generally involves the creation of an electrical field at the surface of a liquid. The resulting electrical forces create a jet of liquid which carries electrical charge. These electrically charged jets of liquid may be attracted to a body or other object at a suitable electrical potential. As the liquid jet is forced farther and farther toward the object, it elongates. As it travels away from the liquid reservoir, it steadily dries and hardens, thereby forming a fiber. The drying and hardening of the liquid jet into a fiber may be caused by cooling of the liquid, i.e., where the liquid is normally a solid at room temperature; evaporation of a solvent, e.g., by dehydration, (physically induced hardening); or by a curing mechanism (chemically induced hardening). Heretofore, the fibers produced by electrospinning techniques have necessarily been collected on a suitably located charged receiver and subsequently removed from it as needed.

Fibers produced by this process have been used in a wide variety of applications, but no such fibers have ever been produced for use as skin care masks. Generally, electrospun fibers are known, from U.S. Patent Nos. 4,043,331 and 4,878,908, to be particularly useful in forming non-woven mats suitable for use in wound dressings. One of the major advantages of using electrostatically spun fibers in wound dressings, is that very thin fibers can be produced having diameters, usually on the order of about 100 nanometers to about 25 microns, and more preferably, on the order of about 500 nanometers to about 5 microns. Thus, these fibers can be collected and formed into non-woven mats of any desired shape and thickness. It will be appreciated that, because of the very small diameter of the fibers, a mat with very small interstices and a high surface area per unit mass, two characteristics that are important in determining the porosity of the mat, can be produced. Still further, U.S. Patent Nos. 4,043,331 and 4,878,908 suggest that such dressings have the advantage that they are usually sufficiently porous to allow interchange of oxygen and water vapor between the atmosphere and the surface of a wound.

Besides providing variability as to the diameter of the fibers or the shape, thickness, or porosity of any non-woven mat produced therefrom, the ability to electrospin the fibers also allows for variability in the composition of the fibers, their density of deposition and their inherent strength. By selectively choosing the composition of the fibers being electrospun, it will be appreciated that fibers having different physical or chemical properties may be obtained. This can be accomplished either by spinning a liquid containing a plurality of components, each of which may contribute a desired characteristic to the finished product, or by simultaneously spinning, from multiple liquid sources, fibers of different compositions that are then simultaneously deposited to form a mat. The resulting wound dressing mat, of course, would consist of intimately intermingled fibers of different material. A further alternative noted in the U.S. patents is to produce a wound dressing mat having a plurality of layers of different fibers of different materials (or fibers of the same material but different characteristics, e.g. diameter), as by, for example, varying with time the fibers being deposited on the receiver.

Thus, U.S. Patent Nos. 4,043,331 and 4,878,908 make it clear that strong, non-woven mats comprising a plurality of fibers of organic, namely polymeric, material produced by electrostatically spinning the fibers from a liquid consisting of the material or precursor thereof is known in the art for use as wound dressings. However, these fibers must necessarily be collected on a suitably charged receiver and subsequently removed therefrom. While U.S. Patent No. 4,043,331 in particular has attempted to provide an improved wound dressing for use on a surface wound by providing a strong, non-woven mat of electrospun, polymeric fibers, it essentially treats the electrospun fibrous mat formed like a piece of gauze used in bandage to protect a surface wound and does not offer any application advantage over traditional non-electrospun fiber bandages other than those advantages provided by the use of such small diameter, electrospun fibers as noted hereinabove. There is also no reference to any other applications for this fibrous mat other than for medical applications such as wound dressings or vascular stents.

Current skin care masks are generally applied as topical creams, lotions or ointments to deliver medically useful additives. Some masks may include dusts or liquid sprays. No such skin masks use nanofibers to deliver these additives however.

More importantly, dusts and liquid sprays may be more likely than fibrous materials to migrate into sensitive areas of the body such as the nose and eyes where the skin mask is being applied to the face.

While attempts have been made heretofore to provide wound dressing and other medical devices from electrospun fibers, the art has not suggested other desired and needed applications. Nor has the art suggested how to directly apply the electrospun fibers directly to the skin without intermediate fabrication steps such as using a transient, charged receiver to first collect the fibers into a mat prior to applying them to the wound.

Therefore, the need continues to exist for a cosmetic skin mask of electrospun fibers that can be applied gently and painlessly as well as directly to the skin to provide healing or skin care treatment to the skin.

WO 98/03267 discloses a device which in one embodiment may deposit fibers on a wound by means of an electrospinning device. Biologically active ingredients may be incorporated.

Ru 2 034 534 discloses a method of applying a coating to skin or a wound by means of a device that incorporates an electrospinning device but in which the liquid to be polymerised is provided in a pressurised container.

### SUMMARY OF INVENTION

It is, therefore, an object of the present invention to provide an improved skin care mask for the treatment of the skin , comprising one or more fibers that are electrostatically spun to form the mask,
wherein the fibers are applied directly to the skin in a more gentle and painless manner.

It is yet another object of the present invention to provide an improved skin mask, as above, which can incorporate various medically useful, skin care additives into the fibers forming the mask.

It is still another object of the present invention to provide an improved skin care mask, as above, which can be readily applied in any size, shape or thickness desired.

It is a further object of the present invention to provide an improved non-therapeutic method for treating certain skin conditions, wherein fibers forming skin masks are directly applied to the skin.

It is a further object of the present invention to provide a non-therapeutic method for treating a skin condition, as above, wherein the skin mask produced is readily peelable or removable.

It is still another object of the present invention to provide a non-therapeutic method for treating a skin condition, as above, wherein the skin mask is capable of removing moisture or oils from the skin.

At least one or more of the foregoing objects, together with the advantages thereof over the known art relating to electrostatic fiber spinning and skin care products, which shall become apparent from the specification that follows, are accomplished by the invention as hereinafter described and claimed.

According to a first aspect of the invention there is provided a mask for affecting a skin condition having the features of claim 1.

In general, the present invention provides a skin mask for affecting a skin condition comprising a fibrous membrane comprising one or more fibers that have been electrostatically spun and applied directly onto the three-dimensional topography of the skin to form the membrane. That is, the electrospun fibers of the present invention are not first collected on a suitably charged receiver and subsequently stripped therefrom, but rather are applied directly to the topography of the skin, providing for a more gentle and painless manner of application. Furthermore, the fibers have been found to be capable of including both soluble and insoluble additives therein. Thus, medically useful skin care additives may be incorporated in the liquid forming the fibers, either as a solution or a dispersion. The electrospun fibers are applied directly to the surface of the skin to form a protective layer on areas of the skin to be treated or protected.

According to a second aspect of the invention there is provided a non-therapeutic method for treating a skin condition an set out in claim 12.

The method comprises the steps of electrostatically spinning one or more fibers and directly assembling the electrospun fibers onto the skin in the form of a skin covering or mask without the use of a transient, charged receiver or other intermediate fabrication step. For those fibers containing medically useful additives, the additives may leach, diffuse, or be otherwise transferred to the skin and, after the additives have performed their function(s), the covering or mask may be removed as by peeling or other removal methods from the skin.

### EMBODIMENT FOR CARRYING OUT THE INVENTION

As noted hereinabove, the present invention is directed toward a cosmetic skin covering or mask containing a poly(ethylenimine) polymer and, optionally, one or more medically useful additives for protecting and/or healing the skin. The skin mask is generated on the skin by directly electrospinning one or more polymeric fibers onto the skin surface. These very thin fibers (i.e., nanofibers) containing the additives provide a larger surface area per unit mass for delivering the additives to the skin than would conventional creams, lotions or ointments, and can deliver the additives in a painless manner. The mask may remain on the skin for a period of time as desired to allow the additives to function, or the additves may be moisture activated with the application of a moist, warm cloth. After the additives have performed their function, the mask may be peeled or otherwise removed from the skin. Importantly, the addition of the additives to the liquid to be electrospun into the fiber (i.e., the spin dope) does not prevent the formation of a covering for the skin using electrospinning techniques.

The polymeric covering or skin mask formed is formulated from one or more fibers consisting of a poly(ethylenimine) polymer. Polyethylenimine (PEI) will form salts with α-hydroxy acids currently used in many skin care products. Therefore, spun salts of PEI will function as a delivery system for these acids to the skin. Of course, other additives as set forth hereinbelow may instead of or with the α-hydroxy acids.

As suggested hereinabove, other additives, either soluble or insoluble, may also be included in the liquid(s) to be electrospun into the fibers. Preferably, these additives are medically important topical additives provided in at least effective amounts for the treatment of the skin. Such amounts depend greatly on the type of additive and the physical properties of the polymer(s) employed, and skin condition to be treated. Generally, however, such additives can be incorporated in the fibers in amounts ranging from trace amounts (less than 0.1 parts by weight per 100 parts polymer) to 500 parts by weight per 100 parts polymer, or more. Examples of such additives include, but are not limited to, asprin; α-hydroxy acids including lactic acid and glycolic acid; retinoids, including all tnans-retinol, etc.; DNA proteins, synthetic polypeptides, vitamin E; fragrances for use in aroma therapy and the like; and oil absorbing polymers such as those commercially available under the tradename Polytrap. Other additives such as oils, soaps, or medication for the treatment of any of a number of skin conditions such as acne or various rashes, can be applied broadly or in patterns to provide additional treatment to selected areas of the skin. Antioxidants and antimicrobial additives as well as analgesics such as lidocaine, soluble or insoluble antibiotics such as neomycin, thrombogenic compounds, nitric oxide releasing compounds such as sydnonimines and NO-complexes that promote skin healing may also be employed in the present invention. Still other additives include carvone and insoluble additives such as waterlok and cellulose. Insect repellants and other physical barriers to insects or bugs may also be incorporated.

Prior studies have shown that PEI, when used in non-fiber form, may stabilize all trans retinol and protect the skin from photooxidation. It is believed that the same characteristics can be found using electrospun fibers. Both retinol and antioxidant activity is welcome in the treatment of the skin. Essentially any additive suitable for the treatment of any of a number of skin conditions wherein additives added to the skin are likely to aid in such treatment or wherein the absorption of excess moisture or oils from the skin is likely to aid in such treatment, can be employed.

It will be appreciated that the fibers can produce a number of different types of coverings or masks depending upon how the fibers are produced. For instance, the liquid to be electrospun into the plurality of fibers may be a mixture of a poly(ethylenimine) polymer and one or more skin care or treatment additives. Thus, one fluid could provide the entire mask. However, it is also envisioned that fibers of different compositions could be spun together or in sequential layers to provide a suitable mask of one or more layers.

As discussed earlier, one of the major advantages of using electrospun fibers, is that these fibers can be produced having very small diameters, usually on the order of about 100 nanometers to about 5 micron, and more preferably, on the order of about 100 nanometers to about 1 micron. Thus, given that these "nanofibers" can be formed into non-woven masks of any desired shape and thickness relatively rapidly, their usefulness and desirability as skin care masks can readily be appreciated.

Because of the very small diameter of the fibers, a mask with very small interstices and high surface area per unit mass is produced. It will be appreciated that the higher surface area allows for far greater utilization and quickens the rate of transfer of the additive(s) to the skin which thereby benefits from the full potential of the additive. The electrospun polymeric masks of the present invention have high surface areas of preferably at least 5 m²/g, and more preferably, approximately 100 m²/g for efficient fluid absorption and dermal delivery.

The electrospun fiber-formed masks of the present invention are lightweight, may be oxygen and moisture permeable, and resistant against airborne contaminants such as dust, microbes, or other infectious agents which might be carried by the air currents or otherwise affect the care of the skin. The ability of the fibers to transport and deliver medically useful additives to the skin can be controlled through the density and thickness of the applied mask, and/or layering of different fiber compositions.

With respect to the fibers, it will be understood that the fibers should preferably be dry so as to have sufficient strength in forming the mask. However, in some instances, a wet fiber may be employed. Such wet fibers are generally softer and conform to the surface of the skin better. In any event, the ability to electrospin very thin fibers (i.e., nanofibers) directly onto the skin surface and to place medically useful additives into the solutions used to create the fibers, thereby incorporating the additives into the polymeric fibers, are very important advances leading to the present invention. The polymer(s) or additive(s) employed in fiber(s) may directly absorb excess moisture or oils from the skin. Moreover, the direct application of the fibers means that the fibers can be advantageously placed in intimate contact with the skin, enabling efficient removal of dead cells, fluid or bacteria from the skin. Direct contact with the surface of the skin will also enable improved additive delivery to the skin. Finally, it will be appreciated that direct application provides for improved and, in fact, inherent sterility of the fibers.

Suitable solvents include acetone, tetrahydrofuran, ethanol and water. It will further be appreciated that the skin treating additives may be soluble or insoluble in the solvent employed. Where the additives are insoluble, they may be encapsulated within the fibers using the electrospinning techniques described. It has been found that fibers produced from these electrospinning techniques with such additives still impart the desired properties of the additive and yet maintain the porous membrane-like properties of the nanofibrous skin mask as described.

It will further be appreciated that essentially any device known in the art capable of electrospinning fibers may be used to electrospin the fibers and deliver the fibers to the skin in forming the skin mask. Preferably, the device will be portable and handheld. One such device is described in a provisional application filed on the same day of this application.

Thus it should be evident that the skin mask and methods for treatment of certain skin conditions as set forth in the present invention are highly effective in protecting and treating the skin and certain conditions thereof. The invention is particularly suited for use as cosmetic facial masks, but is not necessarily limited thereto. For example, other applications may include the use of the membranes or masks for skin beautification for such things as general cosmetics, coverage of burns, scars, scratches, bruises, moles, tattoos, fill indentations or dimples. The product may further be used to cover cellulite, acne, scars, cuts, cysts, or to hide varicose veins, dry skin spots, surgical stitches. The mask and method of the present invention can further be used separately with other equipment methods or, as well as for the manufacture of other skin masks and related materials. For example, electrospun fibers of appropriate composition may be used to provide protection of the skin against other threats such as "last ditch" protection against toxic substances such as chemical warfare agents.

In addition, it is envisioned that the skin masks of the present invention may be used to color or tone the skin such as by coloring or tinting the fibers for general skin cosmetic use. The coloring of the fibers could also be used to provide novelty or specialty coloring to the skin such as "glow in the dark" or fluorescent coloring.

The size and shape of the mask or membrane can be readily controlled, and it is further envisioned that the shape of the mask may be controlled or predetermined via the use of templates and the like as is known in the art with other skin creams.

Based upon the foregoing disclosure, it should now be apparent that the use of the skin mask as described herein will carry out the objects set forth hereinabove.

Skin masks produced according to the present invention are not necessarily limited to those described in any particular embodiment.

## Claims

1. A mask for affecting a skin condition, the mask comprising:
a fibrous membrane comprising one or more electrospun fibers, wherein the one or more fibers arse applied directly onto the three-dimensional topography of the skin to form the membrane wherein the mask or membrane has a surface area per unit mass of at least 5m²/g and **characterised in that** the one or more fibers are formed from a solution of a poly (ethylenimine) polymer and a solvent selected from acetone, tetrahydrofuran, ethanol or water.

2. The mask according to claim 1, wherein the fibers are coloured.

3. The mask according to claim 1, wherein the membrane has a predetermined shape.

4. The mask according to claim 1, wherein the polymer is biodegradable and will at least partially absorb moisture from the skin.

5. The mask according to claim 1, wherein the electrospun fibers include an additive for conditioning or protecting the skin.

6. The mask according to claim 5, wherein the additive is medically useful for treating the skin.

7. The mask according to claim 5, wherein the additive is selected from the group consisting of asprin, α-hydroxy acids, retinoids, DNA proteins, synthetic polypeptides, vitamin E, fragrances, oil absorbing polymers, oils, soaps, antioxidants, antimicrobial additives, insect repellents, and cosmetic additives.

8. The mask according to claim 5, wherein the additive is incorporated into the one or more fibers in amounts ranging from trace amounts to 500 parts by weight per 100 parts polymer.

9. The mask according to claim 1, further comprising a second fibrous membrane forming a layer over the original fibrous membrane.

10. The mask according to claim 1, wherein the fibers have diameters ranging from about 100 nanometers to about 5 microns.

11. The mask according to claim 1, wherein the additives may be absorbed into the skin.

12. A non-therapeutic method for treating a skin condition comprising:
electrostatically spinning one or more fibers to form a mask or membrane and directly assembling the electrostatically spun fibers onto the skin without the use of a transient, charged receiver; wherein the mask or membrane has a surface area per unit mass of at least 5m²/g and **characterised in that** the fibers are formed from a solution of poly (ethylenimine) polymer and a solvent selected from acetone, tetrahydrofuran, ethanol or water.

13. The method according to claim 12, wherein the step of spinning includes the step of forming a fiber from a solution of poly (ethylenimine) polymer and one or more additives for the treatment of the skin.

14. The method according to claim 13, wherein the additives are selected from the group consisting of fragrances, oil absorbing polymers, oils, soaps, and cosmetic additives.

15. A method according to claim 12 comprising covering one of bruises, moles, tattoos, acne, cellulite, cysts, varicose veins, dry skin spots and stitches with the mask or membrane.

## Patentansprüche

1. Maske zum Beeinflussen eines Hautzustands, wobei die Maske aufweist:
eine Fasermembran, die eine oder mehrere elektrogesponnene Fasern aufweist,
wobei die eine oder die mehreren Fasern direkt auf die dreidimensionale Topographie der Haut aufgebracht werden, um die Membran zu bilden, wobei die Maske oder Membran eine Oberflächenfläche pro Masseneinheit von mindestens 5 m²/g hat und **dadurch gekennzeichnet ist, dass** die eine oder die mehreren Fasern aus einer Lösung eines Poly(ethylenimin)-Polymers und eines Lösungsmittels, das aus Aceton, Tetrahydrofuran, Ethanol oder Wasser ausgewählt ist, gebildet werden.

2. Maske nach Anspruch 1, wobei die Fasern farbig sind.

3. Maske nach Anspruch 1, wobei die Membran eine vorbestimmte Form hat.

4. Maske nach Anspruch 1, wobei das Polymer biologisch abbaubar ist und zumindest teilweise Feuchtigkeit von der Haut absorbieren wird.

5. Maske nach Anspruch 1, wobei die elektrogesponnenen Fasern einen Zusatz enthalten, um die Haut in einen gewünschten Zustand zu bringen oder zu schützen.

6. Maske nach Anspruch 5, wobei der Zusatz zum medizinisch behandeln der Haut brauchbar ist.

7. Maske nach Anspruch 5, wobei der Zusatz ausgewählt ist aus der Gruppe, die aus Aspirin, α-Hydroxysäuren, Retinoiden, DNA-Proteinen, synthetischen Polypeptiden, Vitamin E, Duftstoffen, Öl absorbierenden Polymeren, Ölen, Seifen, Antioxidantien, antimikrobiellen Zusätzen, Insekten-Abwehrmitteln und kosmetischen Zusätzen besteht.

8. Maske nach Anspruch 5, wobei der Zusatz in die eine oder die mehreren Fasern in Mengen, die von Spurenmengen bis zu 500 Gew.-Teilen pro 100 Teilen Polymer reichen, inkorporiert ist.

9. Maske nach Anspruch 1, außerdem aufweisend eine zweite Fasermembran, die über der ursprünglichen Fasermembran eine Schicht bildet.

10. Maske nach Anspruch 1, wobei die Fasern Durchmesser haben, die von etwa 100 nm bis etwa 5 µm reichen.

11. Maske nach Anspruch 1, wobei die Zusätze in die Haut absorbiert werden können.

12. Nicht therapeutisches Verfahren zur Behandlung eines Hautzustands aufweisend:
elektrostatisch Spinnen einer oder mehrerer Fasern, um eine Maske oder Membran zu bilden, und unmittelbar Zusammenfügen der elektrostatisch gesponnenen Fasern auf der Haut ohne Verwendung eines geladenen Zwischenaufnehmers; wobei die Maske oder Membran eine Oberflächenfläche pro Masseneinheit von mindestens 5 m²/g hat und **dadurch gekennzeichnet ist, dass** die Fasern aus einer Lösung von Poly(ethylenimin)-Polymer und eines Lösungsmittels, das ausgewählt ist aus Aceton, Tetrahydrofuran, Ethanol oder Wasser, gebildet werden.

13. Verfahren nach Anspruch 12, wobei der Schritt des Spinnens den Schritt des Bildens einer Faser aus einer Lösung von Poly(ethylenimin)-Polymer und einem oder mehreren Zusätzen zur Behandlung der Haut umfasst.

14. Verfahren nach Anspruch 13, wobei die Zusätze ausgewählt werden aus der Gruppe, die aus Duftstoffen, Öl absorbierenden Polymeren, Ölen, Seifen und kosmetischen Zusätzen besteht.

15. Verfahren nach Anspruch 12, aufweisend ein Bedecken mit der Maske oder Membran eines der Folgenden: blaue Flecke, Leberflecke, Tätowierungen, Akne, Cellulitis, Zysten, Krampfadern, trockene Hautflecken und Stiche.

## Revendications

1. Masque permettant de traiter un état de la peau, lequel masque comprend une membrane fibreuse qui comporte des fibres d'électrofilage d'une ou plusieurs sortes, lesquelles fibres d'une ou plusieurs sortes sont appliquées directement sur la structure tridimensionnelle de la peau pour constituer la membrane, le masque ou la membrane présentant une aire par unité de masse d'au moins 5 m²/g, et lequel masque est **caractérisé en ce que** les fibres d'une ou plusieurs sortes sont formées à partir d'une solution d'un polymère poly(éthylène-imine) dans un solvant choisi parmi l'acétone, le tétrahydrofurane, l'éthanol et l'eau.

2. Masque conforme à la revendication 1, dans lequel les fibres sont colorées.

3. Masque conforme à la revendication 1, dans lequel la membrane présente une forme prédéterminée.

4. Masque conforme à la revendication 1, dans lequel le polymère est biodégradable et absorbe, au moins en partie, l'humidité de la peau.

5. Masque conforme à la revendication 1, dans lequel les fibres d'électrofilage contiennent un adjuvant servant à conditionner ou protéger la peau.

6. Masque conforme à la revendication 5, dans lequel l'adjuvant sert à un traitement médical de la peau.

7. Masque conforme à la revendication 5, dans lequel l'adjuvant est choisi dans l'ensemble constitué par les suivants : aspirine, alpha-hydroxy-acides, rétinoïdes, ADN, protéines, polypeptides synthétiques, vitamine E, parfums, polymères oléo-absorbants, huiles, savons, anti-oxydants, adjuvants anti-microbiens, insectifuges, et adjuvants cosmétiques.

8. Masque conforme à la revendication 5, dans lequel l'adjuvant est incorporé dans les fibres d'une ou plusieurs sortes en une quantité allant de traces à 500 parties en poids pour 100 parties de polymère.

9. Masque conforme à la revendication 1, qui comporte en outre une deuxième membrane fibreuse formant une couche par-dessus la membrane fibreuse initiale.

10. Masque conforme à la revendication 1, dans lequel les fibres présentent des diamètres qui valent d'environ 100 nm à environ 5 µm.

11. Masque conforme à la revendication 1, dans lequel les adjuvants peuvent être absorbés par la peau et y pénétrer.

12. Procédé non-thérapeutique de traitement d'un état de la peau, lequel procédé comporte le fait de produire par électrofilage des fibres d'une ou plusieurs sortes pour en faire un masque ou une membrane, et le fait d'assembler ces fibres d'électrofilage directement sur la peau, sans avoir recours à un récepteur chargé transitoire, le masque ou la membrane présentant une aire par unité de masse d'au moins 5 m²/g, et lequel procédé est **caractérisé en ce que** les fibres d'une ou plusieurs sortes sont formées à partir d'une solution d'un polymère poly(éthylène-imine) dans un solvant choisi parmi l'acétone, le tétrahydrofurane, l'éthanol et l'eau.

13. Procédé conforme à la revendication 12, dans lequel l'étape de filage comporte le fait de former des fibres à partir d'une solution d'un polymère poly(éthylène-imine) et d'un ou plusieurs adjuvants servant à un traitement de la peau.

14. Procédé conforme à la revendication 13, dans lequel les adjuvants sont choisis dans l'ensemble constitué par les parfums, polymères oléo-absorbants, huiles, savons, et adjuvants cosmétiques.

15. Procédé conforme à la revendication 12, qui comporte le fait de recouvrir, avec le masque ou la membrane, des ecchymoses, des grains de beauté, des tatouages, de l'acné, de la cellulite, des kystes, des veines variqueuses, des taches cutanées sèches ou des points de suture.
